(19) Europäisches Patentamt
European Patent Office
Office européen des brevets

(11) **EP 3 433 341 B1**

(12) **EUROPEAN PATENT SPECIFICATION**

(45) Date of publication and mention
of the grant of the patent:
**17.03.2021  Bulletin 2021/11**

(21) Application number: **17717886.0**

(22) Date of filing: **17.01.2017**

(51) Int Cl.:
*C10K 1/00* (2006.01)      *C10K 1/02* (2006.01)
*C07C 29/151* (2006.01)    *C10J 3/00* (2006.01)
*C10K 1/20* (2006.01)      *C10K 1/34* (2006.01)
*C10K 3/04* (2006.01)      *C10K 3/06* (2006.01)

(86) International application number:
**PCT/IT2017/000005**

(87) International publication number:
**WO 2018/134853 (26.07.2018 Gazette 2018/30)**

(54) **A PROCESS AND RELATING APPARATUS TO MAKE PURE BIO- METHANOL FROM A SYNGAS ORIGINATED FROM WASTES GASIFICATION**

PROZESS UND VORRICHTUNG ZUR HERSTELLUNG VON REINEM BIO-METHANOL AUS EINER SYNGAS AUS ABFALLVERGASUNG

PROCÉDÉ ET APPAREIL POUR LA FABRICATION DE BIO-METHANOL PUR À PARTIR DE SYNGAS ISSU DE LA GASÉIFICATION DE DÉCHET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.01.2019  Bulletin 2019/05**

(73) Proprietor: **NextChem S.p.A.**
**00156 Rome (IT)**

(72) Inventors:
• **IAQUANIELLO, Gaetano**
**00156 - Rome (IT)**
• **SALLADINI, Annarita**
**00156 - Rome (IT)**

(74) Representative: **Sarpi, Maurizio et al**
**Studio Ferrario S.r.l.**
**Via Collina, 36**
**00187 Roma (IT)**

(56) References cited:
**US-A- 4 110 359          US-A1- 2010 051 875
US-A1- 2011 124 749    US-A1- 2011 160 313
US-A1- 2015 141 719**

**Description**

A process and apparatus for producing a carbon monoxide and hydrogen rich mixture from a syngas generated by the high temperature gasification of municipal, agricultural or industrial derived wastes are disclosed. The process is able to make a mixture which can be converted into methanol.

Field of disclosure

The present disclosure relates generally to the conversion of waste materials into chemicals and/or nitrogen based fertilizers. More specifically, the dsiclosure provides for methods, systems and apparatus for the purification and modification of a syngas generated by high temperature gasification of waste materials as municipal wastes, refuse derived fuel or industrial wastes to make methanol.

Background

Global climate change from one side and growing production of wastes on a worldwide basis from the other, are pushing for questing of new carbon neutral process where wastes can be used as feedstock in alternative to natural gas or other hydrocarbons (bio-fuels).
Such processes will have also advantages over bio-diesel and ethanol production because they are not going to compete for farmland that may be needed for food production.
Processes for the production of synthesis gas (syngas) are known in the art. For example, WO2011/008236 A2 discloses the use of plasma gasification or pyrolysis for production of syngas followed by the production of the hydrogen from syngas in a water gas shift (WGS) reactor.
WO 2009/091325 A1 discloses a biomass gasification method and apparatus for production of syngas with rich hydrogen content; and US Pat n° 5,584,255 discloses a method and apparatus for gasifying organic materials. US Pat n° 6,958,136 discloses a process for the treatment of nitrogen-containing wastes streams that can generate syngas from carbon sources using alkaline metals and carbon radical formation.
WO 2008130260 A1 discloses a waste to liquid hydrocarbon refinery system designed to convert municipal and industrial wastes, biomass and other carbon-containing feedstock into diesel, gasoline and other products. The system involves a high temperature liquid iron bed that generates row syngas from solid and liquid feedstocks and a very high temperature plasma to convert contaminants in the row syngas into ions.
US Pat. N° 6,455,011 B1 discloses a method and apparatus for treating wastes into two-stage gasification which recovers metals or ash content in the wastes in such a state that they can be recycled and gases containing carbon monoxide (CO) and hydrogen gas ($H_2$) for use as synthesis gas for ammonia ($NH_3$) or the other chemicals production.
CN103242134 discloses an invention related to household garbage treatment method, where the waste is subjected to a thermal cracking to form mixed combustible gases containing CO, $CO_2$, $H_2$, nitrogen ($N_2$) and inert argon (Ar) by a full gasification process. The syngas is purified and separated, and then are used for synthesizing methanol and/or dimethyl eter (DME), generating power and synthesizing urea. The system produces significant amounts of tar which be disposed or used. US Pat. Number 2014/0364517 A1 discloses a process and system for producing liquid and gas fuels and other useful chemicals from carbon containing source materials comprises cool plasma gasification and/or pyrolysis to produce syngas which in turn could be used for producing hydrocarbon, methanol, ammonia, urea and other products. The system is carbon neutral but is relatively complex and expensive, requires relatively large amount of energy for the plasma gasification and has quite high cost for maintenance. There remains, therefore, a need for carbon neutral process, apparatus and system for producing bio-fuels as bio-methanol from carbon containing wastes as municipal, RDF and industrial wastes. RDF , a refuse derived fuel , is a fuel produced by shredding and dehydrating solid waste (MSW) with a Waste converter technology. RDF consists largely of combustible components of municipal waste such as plastics and biodegradable waste.
US 2011/160313 discloses a method for improving the overall carbon conversion efficiency of a gasification process, wherein an organic feedstock, which is agricultural or animal waste, introduced into a gasifier, is converted in syngas that is, in turn. utilized for a chemical production process in a chemical production reactor. This invention provides an improved carbon conversion efficiency by recycling the methane-rich purge gas back to the gasification reactor where the methane-rich purge gas back is converted to syngas via the steam -methane reforming reaction. Additionally, there remains a need for a process, apparatus and system that has minimal or zero emissions, in particular relevant to $CO_2$ emissions.

Summary of the invention

The invention is according to the appended claims.
According to one aspect of the disclosure, a process and apparatus for producing a $CO/H_2$ rich mixture is provided in which the process comprises a plurality of high temperature converters and corresponding-pre-treatment units (at least two or better three or four; depending on the lout capacity), followed by a treatment process of the raw syngas in a single line to further purify and re-adjust the $CO/H_2/CO_2$ content to convert it further in methanol.
The inventors started from the observation that the syngas produced by high temperature gasification of untreated or treated municipal or industrial or agricultural wastes contains few contaminants and its R ratio, with

$$R = \frac{H_2\ \%vol - CO_2\%\ vol}{CO\ \%vol + CO_2\ \%\ vol},$$

(normally ranges from 2 to 2.1) is not aligned to what required for methanol synthesis. Although the syngas composition depends on wastes' composition, we could recognize the following family of components/contaminants:

metallic materials, as iron, lead, chromium, copper and other which are mainly discharged by the bottom of high temperature converter, and other contaminants which include:

  a) Chlorine compounds mainly present as HCl
  b) Nitrogen compounds mainly present as HCN and $NH_3$
  c) Sulphur compounds mainly present as $H_2S$, COS and CS, where the ratio of $H_2S$/COS is normally 10/1 but may be as low as to 2 to 1.

In order to overcome these disadvantages, it is provided a method and apparatus wherein the one portion of the above contaminants are eliminated from the syngas produced by high temperature gasification of untreated or treated municipal or industrial wastes by a first or pretreatment purification process which comprises:

- An acidic scrubbing;
- A basic scrubbing
- A mist removal with a Wet Electrostatic Precipitator (WESP).

A liquid stream is generated throughout such started purification step and further treated into a concentration unit where all contaminants are recovered into a solid form. Once preheated, the resulting syngas is accumulated into an atmosphere storage system from where is compressed to 10-20 barg before adjusting the R ratio eliminating the excess of carbon present into the syngas.

The R ratio adjustment will be carried out by the water gas shift (WGS) which is going to maximize the presence of $H_2$ into the stream and a $CO_2$ removal unit. The removal of S compounds may be done before such adjustment or after "sour" or "sweet" WGS .

An advantage of the present process and apparatus is the ability to realign the R ratio to the proper value for the methanol synthesis.

In a preferred embodiment of the disclosure both the high temperature conversion of waste to syngas and the pretreatment step to remove from raw syngas particulate, bulk of metals, chlorine and $NH_3$ , are made through a plurality of high temperature waste converters and pretreatment units depending of the plant capacity.

The terms "comprises/comprising" where used in this specifications is taken to specify the presence or addition of one or more other features, integers, steps, components or group thereof.

Brief description of the drawings

These and other aspects, features and advantages of which embodiments of the disclosure invention are capable of, will be apparent and elucidated from the following description of the present disclosure, reference being made to the accompanying drawings in which:

- Fig. 1 is a schematic representation of the entire process for making methanol from the syngas produced by wastes High temperature conversion on multiple trains;
- Fig. 2 is a schematic representation of the syngas pre-treatment with each high temperature waste converter according to the present disclosure;
- Fig. 3 and Fig. 4 are schematic representation of the syngas R ratio adjustment with sulphur and $CO_2$ removal according to the "sour" and "sweet" process, according to the present disclosure.

Detailed description of the invention

The invention is as specified in independent claim 1 and claim 5. Specific embodiments of the disclosure are described with reference to the accompanying drawings. These embodiments are provided so that this disclosure will be through and complete, and will fully convey the scope of the disclosure to those skilled in the art.

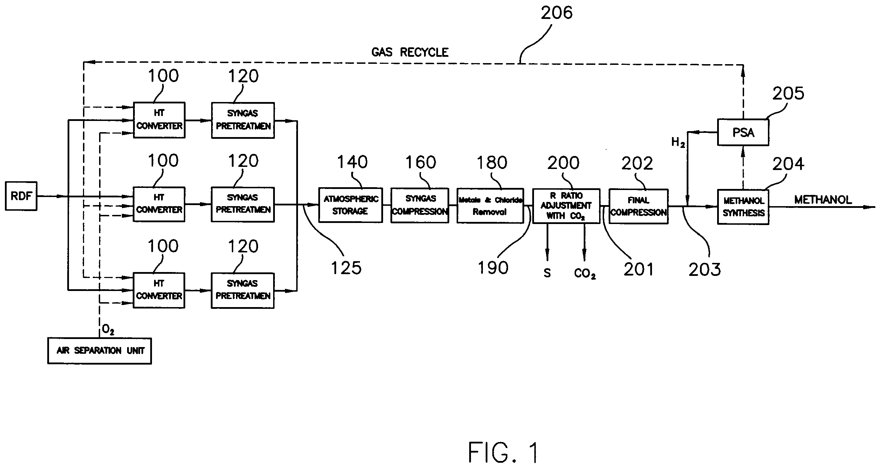

An overview of one embodiment according to the disclosure is shown in fig. 1, when the front end of the process consists of a plurality of converters and pretreatments depending of the plant capacity.

Such plurality will allow:
to reduce the fluctuation of syngas flow and composition to downstream processing;

- to increase the operability time of the entire plant having al least two high temperature waste converters in operation.

According to said embodiment, a process for producing methanol from a syngas produced from a carbon-matrix waste gasification, without any emission, and in particular emission of nitrogen and sulphur, comprises the following steps :

- A high temperature conversion 100 of waste to syngas based on multiple trains;
- A pretreatment step 120 to remove from row syngas particulate, bulk of metals, chlorines and $NH_3$ compounds based also on multiple trains;
- An atmospheric storage 140 ;

- A compression step 160 followed by dechlorine/demetalization reactors 180;
- A syngas R ratio adjustment 200 with sulphur and $CO_2$ removal;
- A final compression 202 of the syngas R ratio adjusted to 70-90 barg for the methanol synthesis 204, from which methanol is obtained;
- A purification step 205 of the purge from the methanol reactor 204 to make pure $H_2$ to be recycled back at the entrance of the same reactor 204 and an off-gas 206 to be recycled back to the high temperature waste converter (100).

A raw syngas coming from one high temperature gasification 100 of organic wastes, or refuse derived fuel (RDF), once cooled in a proper heat recovery boiler or in a quencher is purificated in order to eliminate a portion of the above contaminants in the corresponding pretreatment section 120.

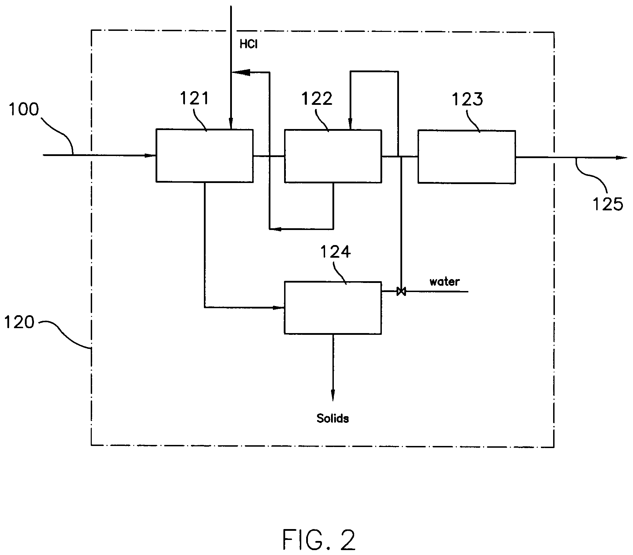

Fig. 2 shows the different steps of the syngas pretreatment 120. Said pretreatment comprises two scrubbing step where, by adding an acidic solution in a column 121 followed by alkaline solution in a column 122 and by a WESP 123, particulate and chlorine compounds are removed in the section 120 and the syngas 190 is ready for conversion, after its compression 160 and the metal and chloride removal 180.

In the conversion step 200 , CO is converted into $CO_2$ and $H_2$ by adding steam; COS is hydrolyzed to $H_2S$, $H_2S$ is reduced to sulphur in a solid form, $CO_2$ is removed via cryogenic unit or an amine unit and pure $CO_2$ is produced.

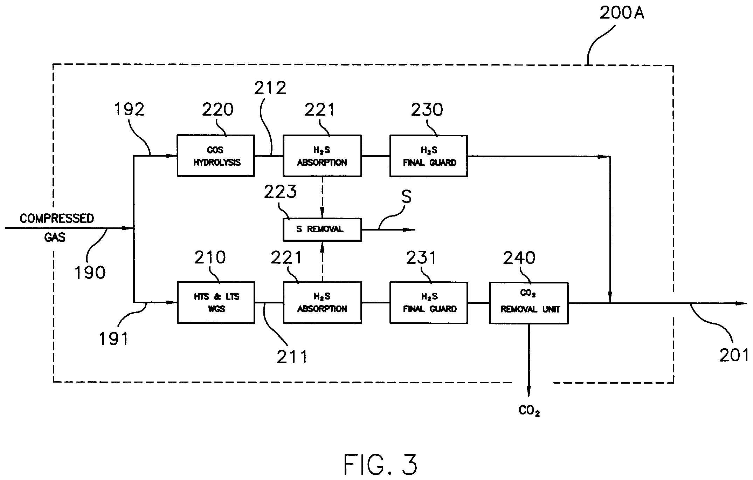

Fig. 3 shows the "sour" water gas shift version 200A of the syngas R ratio adjustment 200, where a portion 191 of the syngas 190 coming from compression 160 is entering a WGS at High and Low temperature to convert the CO with water to $CO_2$ and $H_2$, section 210; this stream enters an absorption section, 222, where $H_2S$ is removed from syngas and transferred into an absorbing liquor in the removal section 223.

The remaining portion 192 of the syngas 190 enters in an hydrolysis reactor 220 where COS is converted to $CO_2$ and $H_2S$, followed by another absorption section, 221, where $H_2S$ is removed and transferred into the absorbing liquor of a removal section 223.

The absorbed liquors from 221 and 222 are treated in 223 to desorb the $H_2S$ and transform it into S. In this section 223 is also present a sulphur recovery unit, where S is separated by the liquor and leaves the unit.

Both streams, 191,192 are further treated to remove $H_2S$ traces through a polishing guard bed, 230/231. The removing of $CO_2$ either with a cryogenic unit or an amine unit, 240, will be carried out only to the portion 193 coming from WGS 210 .

A cryogenic separation of $CO_2$ is required to purify the $CO_2$ before storage.

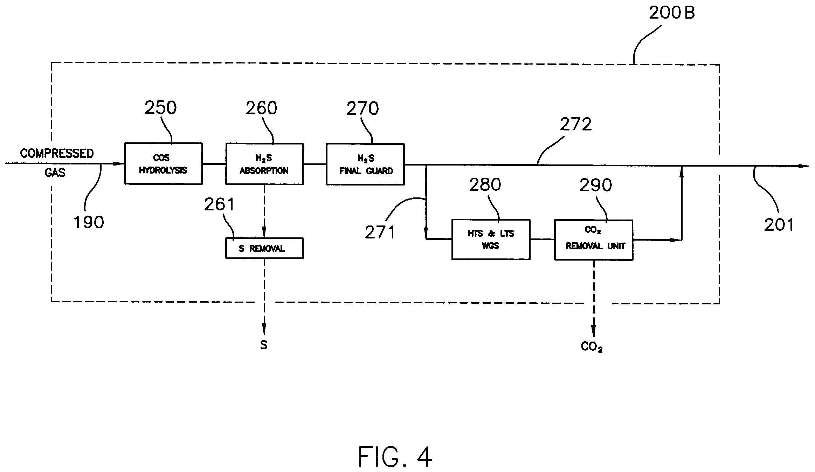

Fig. 4 shows "sweet" water gas shift version 200B of the syngas R ratio adjustment 200, where the syngas 190 coming from compression 160 enters first a COS hydrolyze unit 250 where also HCN is hydrolyzed, followed by $H_2S$ conversion and removal unit, 260, as described before with a final polishing guard bed, 270, to remove all $H_2S$ traces. The syngas leaving such section is split in two 271 and 272, a portion 271 enters a WGS at high and low temperature, 280, to convert the CO with water to $CO_2$ and $H_2$. On such a stream the $CO_2$ will be removed, 290, before mixing the two streams 271 and 272 in 201 to the final compression 202 and the methanol synthesis 204.

## Claims

1. A process for producing methanol from a syngas generated by the gasification of a carbon matrix waste without any emission of nitrogen and sulphur, comprising the following steps:

   - a high temperature conversion (100) of waste to syngas
   - a pretreatment step (120) to remove from raw syngas particulate, bulk of metals, chlorines and $NH_3$ compounds comprising an acidic scrubbing, a basic scrubbing and a mist removal with a Wet Electrostastic Precipitator (WESP) wherein both the high temperature conversion (100) and the pretreatment (120) are made through a plurality of high temperature converters and a pretreatment unit for each of said high temperature converters;
   - an atmospheric storage (140);
   - a single compression step (160) followed by dechlorine/demetalization reactors (180);
   - a syngas R=($H_2$%vol-$CO_2$%vol)/($CO_2$%vol+$CO_2$%vol) ratio adjustment (200) with sulphur and $CO_2$ removal, wherein said syngas R ratio is adjusted: either in "sour" environment (220A comprising the following steps:

     ▪ splitting of syngas in two streams (191 and 192)
     ▪ a water gas shift step (210) to convert CO with water into $CO_2$ and $H_2$ on one stream (211);
     ▪ an hydrolyser reactor (220) where COS of the streams (192) is converted into $CO_2$ and $H_2S$ (212);
     ▪ remove $H_2S$ on both streams (211 and 212) in two separated $H_2S$ absorption sections (221/222) via an absorbing liquid and then transforming it into elementary sulphur and eliminating from the system, in a S removal section (223);
     ▪ a final polishing step (230/231) on both stream resulting from the $H_2S$ absorption

section (221/222) where traces of $H_2S$ are removed from the syngas;

- a $CO_2$ removal unit, (240), via a cryogenic or amine unit on the stream where WGS (210) was installed; or in "sweet" environment (200B), comprising the following steps:
- a COS and HCN hydrolysis step (250) to convert them into $CO_2$, $H_2S$, CO and $NH_3$;
- a $H_2S$ absorption, (260), and removal step, (261), where all incoming $H_2S$ is removed as elementar S and separated from the gas;
- a final polishing step, (270), where traces of $H_2S$ are removed from the syngas;
- a splitting of syngas into two streams (271,272);
- a WGS (280) to convert incoming CO with water into $CO_2$ and $H_2$;
- a $CO_2$ removal unit,(290), on the same output stream from (280) via a cryogenic or amine unit;

- a final compression (202) of said syngas (201) coming out from (200) to 70-90 barg and the methanol synthesis (204)
- a purification step (205) of the purge from the methanol reactor (204) to make pure $H_2$ to be recycled back at the entrance of the same reactor (204) and an off-gas (206) to be recycled back to the high temperature waste converters (100).

2. A process according to one of the preceding claims where the $H_2S$ level into the syngas is controlled by eventually adding waste containing S into the high temperature waste converter (100).

3. A process according to one of the preceding claims where the $CO_2$ recovered from the syngas is removed with a cryogenic unit and stored in a liquid form.

4. A process according to one of the preceding claims wherein the carbon matrix waste is selected from a solid municipal waste, a derived waste fuel as Refused derived Fuel (Rdf), agricultural waste, urban and/or industrial sludge, biomass, a solid chemical waste and combinations thereof and where the waste composition is such to maintain a $H_2S$ concentration in the syngas high enough to carry a sour CO shift.

5. An apparatus for producing methanol from a syngas generated by the gasification of a carbon matrix waste without any emission of nitrogen and sulphur, **characterized in that** it provided :

a high temperature conversion section (100) of waste to syngas;
- a pretreatment section (120) to remove from the raw syngas particulate and the bulk of metals, chlorine and $NH_3$ compounds comprising an acid scrubber column (121), a basic scrubber column (122) and a WESP (123) wherein the high temperature conversion section (100) and the pretreatment section (120) comprise a plurality of high temperature converter and pretreatments units on multiple trains;
- an atmospheric storage (140);
- a compression section (160) followed by dechlorine/demetalization reactors (180);
- a syngas $R=(H_2\%vol-CO_2\%vol)/(CO\%vol+CO_2\%vol)$ ratio adjustment unit (200) with sulphur and $CO_2$ removal wherein said syngas R ratio adjustment unit (200) comprises :
either sour environment, which comprises:

- means for splitting the syngas (190) in two streams (191) and 192);
- a water shift reactor (210) to convert CO with water into $CO_2$ and $H_2$ on one stream (211);
- an hydrolyser reactor (220) where COS of the streams (212) is converted into $CO_2$ and $H_2S$;
- two separated $H_2S$ absorption sections (221/222) to remove $H_2S$ on both streams (211 and 212) via an absorbing liquid and then transforming it into elementary sulphur and eliminating from the system, in a S removal section (223);
- a final polishing guard bed (230/231) on both stream resulting from the $H_2S$ absorption section (221/222) where traces of $H_2S$ are removed from the syngas; $CO_2$ removal unit, (240), via a cryogenic or amine unit on the stream (211) coming from WSG (210); or

sweet environment, which comprises:

- a COS and HCN hydrolysis unit (250) to convert them into $CO_2$, $H_2S$, CO and $NH_3$;
- a $H_2S$ absorption unit, (260), and removal unit (261), where all incoming $H_2S$ is removed as elementar S and separated from the gas;
- a final polishing guard bed, (270), where all traces of $H_2S$ are removed from the syngas;
- means to split the syngas into two streams (271,272);
- a WGS unit (280), to convert incoming CO with water into $CO_2$ and $H_2$;

■ a $CO_2$ removal unit,(290), on the same output stream from (280) via a cryogenic or amine unit.

- a final compression section (202) of said syngas (201) coming out from (200) to 70-90 barg and the methanol synthesis reactor (204), and
- a purification unit (205) of the purge from the methanol reactor (204) to make pure $H_2$ to be recycled back at the entrance of the same reactor (204) and an off-gas (206) to be recycled back to the high temperature waste converters (100).

6. An apparatus according to the preceding claim wherein the carbon matrix waste is selected from a solid municipal waste, a derived waste fuel as Refused derived Fuel (Rdf), agricultural waste, urban and/or industrial sludge, biomass, a solid chemical waste and combinations thereof and where the waste composition is such to maintain a $H_2S$ concentration in the syngas high enough to carry a sour CO shift.

7. A process according to claims 1 - 4 wherein the $H_2$ required to minimize or avoid any $CO_2$ emission is obtained by water electrolysis or from a conventional methanol plant based on hydrocarbons feedstock.

8. A process according to claims 1 - 4 wherein extra $H_2$ required to minimize or avoid any $CO_2$ emission is produced by electrolysis only when excess of electricity is available from the grid.

**Patentansprüche**

1. Verfahren zur Herstellung von Methanol aus einem Synthesegas, das durch die Vergasung eines Kohlenstoffmatrix-Abfalls erzeugt wird, ohne jegliche Emission von Stickstoff und Schwefel, umfassend die folgenden Schritte:

- eine Hochtemperaturumwandlung (100) des Abfalls in Synthesegas;
- einen Vorbehandlungsschritt (120), um aus dem rohen Synthesegas Partikel, Bulk- Metalle, Chlor und $NH_3$-Verbindungen zu entfernen, der eine saure Aufreinigung, eine basische Aufreinigung und eine Nebelentfernung mit einem elektrostatischen Nassabscheider (WESP) umfasst, wobei sowohl die Hochtemperaturumwandlung (100) als auch die Vorbehandlung (120) durch eine Vielzahl von Hochtemperaturumwandlern und eine Vorbehandlungseinheit für jeden der Hochtemperaturumwandler durchgeführt werden;
- einen atmosphärischen Speicher (140);

- einen einzelnen Verdichtungsschritt (160), gefolgt von Dechlorierungs-/Demetallisierungsreaktoren (180);
- eine Synthesegas-R= ($H_2$%vol-$CO_2$%vol)/(CO%vol+$CO_2$%vol)-Verhältniseinstellung (200) mit Schwefel- und $CO_2$-Entfernung,
wobei das Synthesegas-R-Verhältnis eingestellt wird: entweder in einer "sauren" Umgebung (220A), umfassend die folgenden Schritte:

■ Aufspalten des Synthesegases in zwei Ströme (191 und 192)
■ einen Wassergas-Verschiebungsschritt (210) zur Umwandlung von CO mit Wasser in $CO_2$ und $H_2$ in einem Strom (211),
■ einen Hydrolysereaktor (220), in dem COS der Ströme (192) in $CO_2$ und $H_2S$ (212) umgewandelt wird;
■ Entfernen von $H_2S$ aus beiden Strömen (211 und 212) in zwei getrennten $H_2S$-Absorptionsabschnitten (221/222) über eine Absorptionsflüssigkeit und anschließend Umwandeln dessen in elementaren Schwefel und Entfernen aus dem System, in einem H2S-Entfernungsabschnitt (223);
■ einen abschließenden Polierschritt (230/231) an beiden aus dem $H_2S$-Absorptionsabschnitt (221/222) resultierenden Strömen, in dem Spuren von $H_2S$ aus dem Synthesegas entfernt werden,
■ eine $CO_2$Entfernungseinheit (240) über eine kryogene oder Amin-Einheit auf dem Strom, in dem WGS (210) installiert wurde;

oder in
einer "süßen" Umgebung (200B), umfassend die folgenden Schritte:

■ einen COS- und HCN-Hydrolyseschritt (250), um sie in $CO_2$, $H_2S$, CO und $NH_3$ umzuwandeln;
■ einen $H_2S$-Absorptions- (260) und -Entfernungsschritt (261), wobei das gesamte einströmende $H_2S$ als elementares S entfernt und aus dem Gas abgetrennt wird;
■ einen abschließenden Polierschritt (270), wobei Spuren von $H_2S$ aus dem Synthesegas entfernt werden;
■ ein Aufspalten des Synthesegases in zwei Ströme (271, 272);
■ eine WGS (280), um einströmendes CO mit Wasser in $CO_2$ und $H_2$ umzuwandeln;
■ eine $CO_2$Entfernungseinheit, (290), an dem gleichen Ausgangsstrom von (280) über eine kryogene oder Amin-Einheit;

- eine Endverdichtung (202) des aus (200) kommenden Synthesegases (201) auf 70-90 barg und die Methanolsynthese (204);
- einen Reinigungsschritt (205) des Spülgases aus dem Methanolreaktor (204), um reines $H_2$ herzustellen, das am Eingang desselben Reaktors (204) zurückgeführt wird, und ein Abgas (206), das zu den Hochtemperatur-Abfallumwandlern (100) zurückgeführt wird.

2. Verfahren nach einem der vorhergehenden Ansprüche, wobei der $H_2S$-Gehalt in dem Synthesegas durch letztendliche Zugabe von S-haltigem Abfall in den Hochtemperatur-Abfallumwandler (100) gesteuert wird.

3. Verfahren nach einem der vorhergehenden Ansprüche, wobei das aus dem Synthesegas zurückgewonnene $CO_2$ mit einer kryogenen Einheit entfernt und in flüssiger Form gespeichert wird.

4. Verfahren nach einem der vorhergehenden Ansprüche, wobei der Kohlenstoffmatrix-Abfall ausgewählt ist aus einem festen Siedlungsabfall, einem abgeleiteten Abfallbrennstoff als Refused Derived Fuel (Rdf), landwirtschaftlichem Abfall, städtischem und/oder industriellem Schlamm, Biomasse, einem festen chemischen Abfall und Kombinationen davon, und wobei die Abfallzusammensetzung so beschaffen ist, dass eine $H_2S$-Konzentration im Synthesegas aufrechterhalten wird, die hoch genug ist, um eine saure CO-Verschiebung zu bewirken.

5. Vorrichtung zur Herstellung von Methanol aus einem Synthesegas, das durch die Vergasung eines Kohlenstoffmatrix-Abfalls erzeugt wird, ohne jegliche Emission von Stickstoff und Schwefel, **dadurch gekennzeichnet, dass** sie Folgendes vorsieht:

- einen Hochtemperatur-Umwandlungsabschnitt (100) von Abfall zu Synthesegas;
- einen Vorbehandlungsabschnitt (120), um aus dem rohen Synthesegas Partikel und Bulk- Metalle, Chlor- und $NH_3$-Verbindungen zu entfernen, der eine saure Aufreinigungssäule (121), eine basische Aufreinigungssäule (122) und einen WESP (123) umfasst, wobei der Hochtemperatur-Umwandlungsabschnitt (100) und der Vorbehandlungsabschnitt (120) eine Vielzahl von Hochtemperaturumwandlern und Vorbehandlungseinheiten in mehreren Zügen umfassen;
- einen atmosphärischen Speicher (140),
- einen Verdichtungsabschnitt (160), gefolgt von Dechlorierungs-/Demetallisierungsreaktoren (180);
- eine Synthesegas-R=(H20/0VO|-C020/0VO|)/(CO0/0VO|+C020/0VO|) Verhältnis-Einstellungseinheit (200) mit Schwefel- und $CO_2$Entfernung, wobei die Synthesegas-R-Verhältnis-Einstellungseinheit (200) umfasst:
entweder
eine saure Umgebung, welche umfasst:

▪ Mittel zum Aufspalten des Synthesegases (190) in zwei Ströme (191) und (192);
▪ einen Wasserverschiebungsreaktor (210) zur Umwandlung von CO mit Wasser in $CO_2$ und $H_2$ in einem Strom (211),
▪ einen Hydrolysereaktor (220), in dem COS der Ströme (212) in $CO_2$ und $H_2$ umgewandelt wird;
▪ zwei getrennte $H_2S$-Absorptionsabschnitte (221/222), um $H_2S$ in beiden Strömen (211 und 212) über eine Absorptionsflüssigkeit zu entfernen und dann in elementaren Schwefel umzuwandeln und aus dem System zu entfernen, in einem S-Entfernungsabschnitt (223);
▪ ein abschließendes Polier-Schutzbett (230/231) auf beiden Strömen, die aus dem $H_2S$-Absorptionsabschnitt (221/222) resultieren, wobei Spuren von $H_2S$ aus dem Synthesegas entfernt werden; eine $CO_2$Entfernungseinheit (240), über eine kryogene oder Amin-Einheit auf dem Strom (211), der aus dem WSG (210) kommt;

oder
eine süße Umgebung, welche umfasst:

▪ eine COS- und HCN-Hydrolyseeinheit (250), um sie in $CO_2$, $H_2S$, CO und $NH_3$ umzuwandeln;
▪ eine $H_2S$-Absorptionseinheit (260) und eine Entfernungseinheit (261), in der das gesamte einströmende $H_2S$ als elementares S entfernt und vom Gas getrennt wird;
▪ ein abschließendes Polier-Schutzbett (270), in dem alle Spuren von $H_2S$ aus dem Synthesegas entfernt werden;
▪ Mittel zum Aufteilen des Synthesegases in zwei Ströme (271, 272);
▪ eine WGS-Einheit (280), um einströmendes CO mit Wasser in $CO_2$ und $H_2$ umzuwandeln;
▪ eine $CO_2$Entfernungseinheit (290), an demselben Ausgangsstrom von (280) über eine kryogene oder Amin-Einheit;

- einen Endverdichtungsabschnitt (202) des aus (200) kommenden Synthesegases (201) auf 70-90 barg und den Methanolsynthesereaktor (204), und
- eine Reinigungseinheit (205) des Spülgases aus dem Methanolreaktor (204) zur Herstellung

von reinem $H_2$, das am Eingang desselben Reaktors (204) zurückgeführt wird, und eines Abgases (206), das zu den Hochtemperatur-Abfallumwandlern (100) zurückgeführt wird.

6. Vorrichtung nach dem vorhergehenden Anspruch, wobei der Kohlenstoffmatrix-Abfall ausgewählt ist aus einem festen Siedlungsabfall, einem abgeleiteten Abfallbrennstoff als Refused Derived Fuel (Rdf), landwirtschaftlichem Abfall, städtischem und/oder industriellem Schlamm, Biomasse, einem festen chemischen Abfall und Kombinationen davon, und wobei die Abfallzusammensetzung so beschaffen ist, dass eine $H_2S$-Konzentration im Synthesegas hoch genug ist, um eine saure CO-Verschiebung zu bewirken.

7. Verfahren nach einem der Ansprüche 1 bis 4, wobei das $H_2$, das zur Minimierung oder Vermeidung jeglicher $CO_2$-Emission erforderlich ist, durch Wasserelektrolyse oder aus einer herkömmlichen Methanolanlage auf der Basis von Kohlenwasserstoff-Einsatzmaterial gewonnen wird.

8. Verfahren nach einem der Ansprüche 1 bis 4, wobei zusätzliches $H_2$, das zur Minimierung oder Vermeidung jeglicher $CO_2$-Emission erforderlich ist, durch Elektrolyse nur dann erzeugt wird, wenn ein Überschuss an Elektrizität aus dem Netz verfügbar ist.

## Revendications

1. Procédé de production de méthanol à partir d'un gaz de synthèse généré par la gazéification de déchets à matrice de carbone sans aucune émission d'azote et de soufre, comprenant les étapes suivantes :

  - une conversion haute température (100) de déchets en gaz de synthèse ;
  - une étape de prétraitement (120) pour éliminer la masse de métaux, de chlore et de composés $NH_3$ des particules de gaz de synthèse brut, comprenant un lavage acide, un lavage basique, et une suppression de brouillard à l'aide d'un précipitateur électrostatique humide (WESP),
  dans lequel la conversion haute température (100) et le prétraitement (120) sont tous les deux réalisés par l'intermédiaire d'une pluralité de convertisseurs haute température et d'une unité de prétraitement pour chacun desdits convertisseurs haute température ;
  - un stockage atmosphérique (140) ;
  - une étape de compression unique (160) suivie par des réacteurs de déchloration/démétallisation (180) ;
  - un ajustement (200) du rapport R=($H_2$%vol-

$CO_2$%vol)/(CO%vol+$CO_2$%vol) du gaz de synthèse avec élimination du soufre et du $CO_2$, dans lequel ledit rapport R du gaz de synthèse est ajusté :

  soit dans un environnement « acide » (220A), comprenant les étapes suivantes :

    • la division du gaz de synthèse en deux flux (191 et 192) ;
    • une étape de conversion du gaz à l'eau (210) pour convertir le CO avec de l'eau en $CO_2$ et $H_2$ sur un flux (211) ;
    • un réacteur hydrolyseur (220) où le COS des flux (192) est converti en $CO_2$ et $H_2S$ (212) ;
    • l'élimination du $H_2S$ sur les deux flux (211 et 212) dans deux sections d'absorption de $H_2S$ (221/222) distinctes par l'intermédiaire d'un liquide absorbant et ensuite sa transformation en soufre élémentaire et son élimination du système, dans une section d'élimination de S (223) ;
    • une étape de polissage final (230/231) sur les deux flux résultant de la section d'absorption de $H_2S$ (221/222) où les traces de $H_2S$ sont éliminées du gaz de synthèse ;
    • une unité d'élimination de $CO_2$, (240), par l'intermédiaire d'une unité cryogénique ou amine sur le flux où la conversion du gaz à l'eau (WGS) (210) a été installée ;

  ou dans un environnement « doux » (200B), comprenant les étapes suivantes :

    • une étape d'hydrolyse de COS et de HCN (250) pour les convertir en $CO_2$, $H_2S$, CO et $NH_3$ ;
    • une étape d'absorption du $H_2S$, (260), et d'élimination, (261), où tout le $H_2S$ entrant est éliminé en tant que S élémentaire et séparé du gaz ;
    • une étape de polissage final, (270), où les traces de $H_2S$ sont éliminées du gaz de synthèse ;
    • une division du gaz de synthèse en deux flux (271, 272) ;
    • une WGS (280) pour convertir le CO entrant avec de l'eau en $CO_2$ et $H_2$ ;
    • une unité d'élimination de $CO_2$, (290), sur le même flux de sortie provenant de (280) par l'intermédiaire d'une unité cryogénique ou aminé ;

  - une compression finale (202) dudit gaz de synthèse (201) sortant de (200) à 70-90 barg et la synthèse du méthanol (204) ;
- une étape de purification (205) de la purge du réacteur de méthanol (204) pour amener le $H_2$ pur à être recyclé à l'entrée du même réacteur (204) et un gaz de dégagement (206) à être recyclé vers les convertisseurs de déchets haute température (100).

2. Procédé selon l'une des revendications précédentes où le niveau de $H_2S$ dans le gaz de synthèse est contrôlé en ajoutant à terme des déchets contenant du S dans le convertisseur de déchets haute température (100).

3. Procédé selon l'une des revendications précédentes où le $CO_2$ récupéré depuis le gaz de synthèse est éliminé à l'aide d'une unité cryogénique et stocké sous une forme liquide.

4. Procédé selon l'une des revendications précédentes, dans lequel les déchets à matrice de carbone sont sélectionnés parmi des déchets municipaux solides, un combustible résiduaire comme un combustible dérivé de déchets, des déchets agricoles, des boues urbaines et/ou industrielles, de la biomasse, des déchets chimiques solides ou des combinaisons de ceux-ci, et où la composition de déchets est telle qu'elle maintient une concentration de $H_2S$ dans le gaz de synthèse suffisamment élevée pour réaliser une conversion de CO acide.

5. Appareil de production de méthanol à partir d'un gaz de synthèse généré par la gazéification de déchets à matrice de carbone sans aucune émission d'azote et de soufre, **caractérisé en ce qu'**il comprend :

   - une section de conversion haute température (100) de déchets en gaz de synthèse ;
   - une section de prétraitement (120) pour éliminer les masses de métaux, de chlore et de composés $NH_3$ des particules de gaz de synthèse brut comprenant une colonne de lavage acide (121), une colonne de lavage basique (122) et un WESP (123),
   dans lequel la section de conversion haute température (100) et la section de prétraitement (120) comprennent une pluralité de convertisseurs haute température et d'unités de prétraitement sur de multiples trains ;
   - un stockage atmosphérique (140) ;
   - une section de compression (160) suivie par des réacteurs de déchloration/démétallisation (180) ;
   - une unité (200) d'ajustement du rapport $R=(H_2\%vol-CO_2\%vol)/(CO\%vol+CO_2\%vol)$ du gaz de synthèse avec élimination du soufre et du $CO_2$ dans lequel ladite unité (200) d'ajustement du rapport R du gaz de synthèse comprend :

   soit un environnement acide, qui comprend :

   • des moyens pour diviser le gaz de synthèse (190) en deux flux (191) et (192) ;
   • un réacteur de conversion du gaz à l'eau (210) pour convertir le CO avec de l'eau en $CO_2$ et $H_2$ sur un flux (211) ;
   • un réacteur hydrolyseur (220) où le COS des flux (212) est converti en $CO_2$ et $H_2S$ ;
   • deux sections d'absorption du $H_2S$ (221/222) distinctes pour éliminer le $H_2S$ sur les deux flux (211 et 212) par l'intermédiaire d'un liquide absorbant et ensuite sa transformation en soufre élémentaire et son élimination du système, dans une section d'élimination de S (223) ;
   • un lit de garde de polissage final (230/231) sur les deux flux résultant de la section d'absorption de $H_2S$ (221/222) où les traces de $H_2S$ sont éliminées du gaz de synthèse ; une unité d'élimination de $CO_2$, (240), par l'intermédiaire d'une unité cryogénique ou amine sur le flux (211) venant de la WGS (210) ;

   ou un environnement doux, qui comprend :

   • une unité d'hydrolyse de COS et de HCN (250) pour les convertir en $CO_2$, $H_2S$, CO et $NH_3$ ;
   • une unité d'absorption de $H_2S$, (260), et une unité d'élimination (261), où tout le $H_2S$ entrant est éliminé en tant que S élémentaire et séparé du gaz ;
   • un lit de garde de polissage final, (270), où toutes les traces de $H_2S$ sont éliminées du gaz de synthèse ;
   • des moyens pour diviser le gaz de synthèse en deux flux (271, 272) ;
   • une unité WGS (280) pour convertir le CO entrant avec de l'eau en $CO_2$ et $H_2$ ;
   • une unité d'élimination de $CO_2$, (290), sur le même flux de sortie depuis (280) par l'intermédiaire d'une unité cryogénique ou amine.

   - une section de compression finale (202) dudit gaz de synthèse (201) sortant de (200) à 70-90 barg et le réacteur de synthèse de méthanol (204), et

- une unité de purification (205) de la purge du réacteur de méthanol (204) pour amener le $H_2$ pur à être recyclé à l'entrée du même réacteur (204) et un gaz de dégagement (206) à être recyclé vers les convertisseurs de déchets haute température (100).

6. Appareil selon la revendication précédente dans lequel les déchets à matrice de carbone sont sélectionnés parmi des déchets municipaux solides, un combustible résiduaire comme un combustible dérivé de déchets, des déchets agricoles, des boues urbaines et/ou industrielles, de la biomasse, des déchets chimiques solides ou des combinaisons de ceux-ci, et où la composition des déchets est telle qu'elle maintient une concentration de $H_2S$ dans le gaz de synthèse suffisamment élevée pour réaliser une conversion de CO acide.

7. Procédé selon les revendications 1 à 4, dans lequel le $H_2$ nécessaire pour minimiser ou éviter toute émission de $CO_2$ est obtenu par électrolyse de l'eau ou à partir d'une installation de production de méthanol conventionnelle basée sur une charge d'alimentation d'hydrocarbures.

8. Procédé selon les revendications 1 à 4, dans lequel le $H_2$ supplémentaire nécessaire pour minimiser ou éviter toute émission de $CO_2$ est produit par électrolyse uniquement lorsqu'un surplus d'électricité est disponible sur le réseau.

FIG. 1

EP 3 433 341 B1

FIG. 2

EP 3 433 341 B1

200A

192   220   212   221   230

COS HYDROLYSIS   H₂S ABSORPTION   H₂S FINAL GUARD

COMPRESSED GAS

190

223

S REMOVAL   S

210   221   231   240

HTS & LTS WGS   H₂S ABSORPTION   H₂S FINAL GUARD   CO₂ REMOVAL UNIT

191

211

201

CO₂

FIG. 3

FIG. 4

# EP 3 433 341 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011008236 A2
- WO 2009091325 A1
- US 5584255 A
- US 6958136 B
- WO 2008130260 A1
- US 6455011 B1
- CN 103242134
- US 20140364517 A1
- US 2011160313 A